# EUROPEAN PATENT SPECIFICATION

(11) **EP 1 628 648 B1**
(45) Date of publication and mention of the grant of the patent: **19.12.2007**
(21) Application number: 04735926.0
(22) Date of filing: 03.06.2004
(51) Int. Cl.: A61K 31/13, A61K 9/20, C07C 217/12, A61P 25/22

(54) **DERAMCICLANE-FUMARATE TABLETS**
DERAMCICLAN-FUMARAT TABLETTEN
COMPRIMES DE DERAMCICLANE-FUMARATE

(30) Priority: 03.06.2003 HU 0301537
(43) Date of publication of application: 01.03.2006
(73) Proprietor: EGIS Gyógyszergyár Nyrt, 1106 Budapest (HU)
(72) Inventor: FEKETE, Pál, H-1147 Budapest (HU); MAROSHELYI, Lászlóné, H-1165 Budapest (HU); ZSIGMOND, Zsolt, H-1173 Budapest (HU); GORA, Lászlóné, H-2117 Isaszeg (HU); LEVENTISZNÉ HUSZAR, Magdolna, H-1125 Budapest (HU); JAMBOR, Istvánné, H-1182 Budapest (HU); TARI, József, H-1172 Budapest (HU)
(74) Representative: Beszédes, Stephan G.
(86) International application number: PCT/HU2004/000058
(87) International publication number: WO 2004/105743

(56) References cited:
- EP-A- 0 694 299
- WO-A-00/37055
- US-A- 4 342 762

## Description

### TECHNICAL FIELD OF THE INVENTION

The invention relates to deramciclane-fumarate tablets of high active ingredient content and a process for the preparation thereof.

### STATE OF THE ART

It is known that (1R,2S,4R)-(-)-2-dimethylaminoethoxy-2-phenyl-1,7,7-trimethyl-bicyclo[2.2.1]heptane-2-(E)-butenedioate (1:1) of the Formula I (referred to furtheron as deramciclane-fumarate) is a valuable anxiolytic pharmaceutical active ingredient (GB 2,065,122; EP 1 052 245).

The preparation of the active ingredient and pharmaceutical compositions containing the same - i.e. tablets - is described in GB 2,065,122.

Deramciclane-fumarate is a white crystalline powder which according to practical experience possesses extremely unfavourable tabletting properties.

The following properties of deramciclane-fumarate are very unfavourable from the point of view of the production of tablets:
- weak cohesion;
- high elasticity;
- strong adhesion;
- low water solubility.

Due to the weak cohesion characteristics of deramciclane-fumarate on compression of the particles of the active ingredient acceptable tablet strength can not be obtained. When filling 300 mg of deramciclane-fumarate powder into a die cavity of 10 mm diameter of an eccentric press machine and thereafter pressing with a compression force of 5-40 kN, the crushing strength of the tablets does not exceed 10 N. In case of suitably compressible materials the attainable crushing strength is higher than 100 N.

The highly elastic behaviour of the particles of the active ingredient means that under the effect of compression force the volume of the deramciclane-fumarate particles is decreased, whereupon when compression is no more exerted, the particles regain their original form to a significant extent and due to said elastic re-conversion the bonds formed during compression among the particles are broken up. Said disruption of the bonds takes place along the maximal force planes formed within the tablet and this causes the lamination of the tablet structure. In case of conveniently compressible materials having low elasticity the internal structure of the tablet is homogenous and no laminate structure is formed.

The low tablet crushing strength and lamination can be eliminated by using a large amount of a binder. However, a large amount of the binder prolongs the disintegration time of the tablet in aqueous medium to an excessive extent and additionally it slows down the dissolution of the active ingredient; accordingly tablets of unsuitable quality are obtained.

Due to the strongly adhesive properties of deramciclane-fumarate on the one hand during compression high friction forces are formed between the side of the tablets and the wall of die, which result in a damage of the tablet when it is removed from the die. On the other hand the tablets stick to the surface of the punches and therefore the surface of the tablet becomes uneven. This drawback can be eliminated in principle by adding a large amount of a lubricant; however, this measure has serious disadvantages; it decreases the strength of the tablets to an undesired extent, due to the hydrophobic character it considerably slows down the disintegration of tablet in aqueous medium, it also slows down the dissolution of the active ingredient and for the reasons stated above tablet of unsuitable quality are obtained.

In addition to the low water solubility of deramciclane-fumarate the weak cohesion properties and strong adhesion are still more problematic because the required larger amount of the binders on the one hand and the lubricants on the other make the fast dissolution of the active ingredient impossible.

Because of the problems discussed above the tablet formulations described in GB 2,065,122 are unsuitable for industrial scale manufacture of deramciclane-fumarate tablets.

The tablet formulations disclosed in GB 2,065,122 are particularly unsuitable for the preparation of deramciclane-fumarate tablets having an active ingredient content above 50 % by weight. However, both the manufacturers and the patients prefer tablets having such a high active ingredient content. From the point of view of the manufacturers the smaller tablet weight requires less auxiliary agents and a shorter labour time, a higher batch size, less analytical tests, i.e. lower manufacturing costs. The patients can swallow smaller tablets more easily and this improves the patient compliance.

EP 694 299 discloses compositions with up to one third of deramciclane as active agent.

### SUMMARY OF THE INVENTION

The object is the invention is the development of deramciclane-fumarate tablets which have an active ingredient content above 50 % by weight and can be readily compressed into tablets.

A further object of the present invention is the elaboration of a process suitable for the preparation of such tablets.

The present invention is directed to tablets comprising deramciclane-fumarate of the Formula 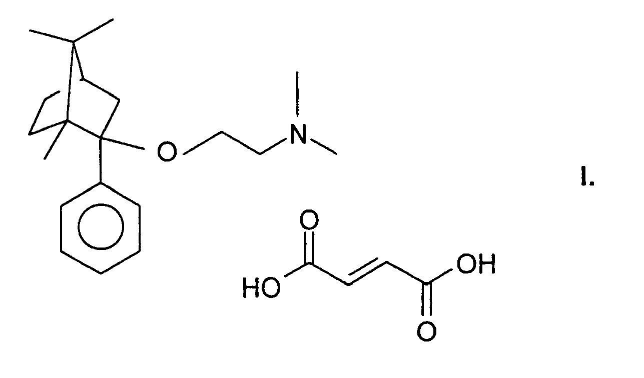 whereby said tablets contain (related to the total weight) more than 50 % by weight of deramciclane-fumarate, 5-20 % by weight of a binder, 5-20 % by weight of microcrystalline cellulose having an average particle size below 30 µm, 1-10 % by weight of a disintegrant, 0.5-4 % by weight of a lubricant, 0.5-4 % by weight of an anti-adhesive agent and 0-30 % by weight of a filler.

### DETAILED DESCRIPTION OF THE INVENTION

The present invention is based on the recognition that if microcrystalline cellulose having a particle size below 30 µm is used in the granulating liquid, the particles of such microcrystalline cellulose form a layer on the surface of deramciclane-fumarate particles and thus decrease the adhesion properties of the granules. Thus the amount of lubricants and anti-adhesive agents required in the tablet can be reduced and in this manner non-sticking tablets having appropriate strength can be manufactured. The tablets according to the present invention possess good mechanical strength, disintegrate easily and quickly in aqueous medium and the active ingredient content is promptly dissolved.

The deramciclane-fumarate content of the tablets according to the present invention is 50-88 % by weight, preferably 50-78 % by weight.

According to a preferred embodiment of the present invention there are provided tablets comprising as active ingredient (I R,2S,4R)-(-)-2-dimethylaminoethoxy-2-phenyl- 1,7,7-trimethyl-bicyclo[2.2.1]heptane-2-(E)-butenedioate (1:1) of the Formula I which contain not more than 0.2 % by weight of (1R,3S,4R)-(-)-3-(2-N,N-dimethylaminoethyl)-1,7,7-trimethyl-bicyclo[2.2.1]heptane-2-one-(E)-butenedioate (1:1) of the Formula

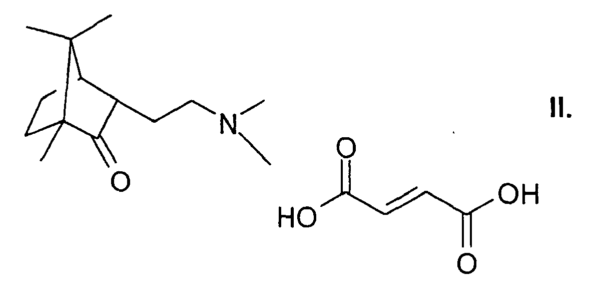

Such high purity deramciclane-fumarate is described in EP 1 052 245.

The deramciclane-fumarate tablets of the present invention can comprise any binder suitable for the purposes of the manufacture of tablets. Such binders are disclosed e.g. in the chapter National Formulary of the US Pharmacopoeia [USP23/NF 18 page 2206 (United States Pharmacopeial Convention, Inc., 1995)]. Thus as binder preferably polyvinyl pyrrolidone, gum-arabic, alginic acid, sodium carboxymethyl cellulose, dextrine, ethyl cellulose, gelatine, liquid sugar, guar gum, hydroxypropyl methyl cellulose, methyl cellulose, polyethylene oxide, pre-gelatinised starch or sugar syrup, particularly polyvinyl pyrrolidone (povidone) or a copolymer of vinyl pyrrolidone and vinyl acetate (co-povidone) can be used.

The tablets according to the present invention contain 5-20 % by weight, preferably 5-12 % by weight, particularly 6-9 % by weight of a binder (related to the total weight of the tablet).

The granulating liquid used for dissolving the binder can be preferably water, ethanol, isopropanol or a mixture thereof containing said solvents in any ratio. Microcrystalline cellulose having a average particle size not higher than 30 µm is suspended in the granulating liquid, whereupon the powder of the active ingredient is granulated with said granulating suspension directly without adding further auxiliary agents. Granulating can be carried out by the wet granulating method whereby the above granulating suspension is added to the powder of the active ingredient in a high shear mixer. According to another procedure granulation is performed by the spraying method whereby the above granulating suspension is sprayed onto the powder of the active ingredient in a fluidization spraying apparatus.

Microcrystalline cellulose having an average particle size of 30 µm or below is generally designated by No. 105. The most frequently used commercially available products are Avicel 105, Vivapur 105 and Elcema 105. The brochures of the manufacturers recommend the use of microcrystalline cellulose of this type as inert carrier, or alternatively as auxiliary agent useful in the manufacture of suspension and suppositories in order to prevent sedimentation.

Nowadays in the manufacture of tablets microcrystalline cellulose having an average particle size of at least 50 µm is used. Microcrystalline cellulose having an average particle size of 50 µm is generally denoted by No. 101, while microcrystalline cellulose having an average particle size of 90-100 µm is generally designated as No. 102.

According to the state of the art various microcrystalline cellulose types are used by admixing microcrystalline cellulose with the active ingredient and optionally with other auxiliary agent in powder form and thereafter subjecting the powder mixture to direct tabletting by compression. If the flowing properties and/or the compressability of the powder mixture are not suitable, the wet granulating method is used whereby the powder mixture is granulated with a solution of a binder and/or the binding agent is used as powder and the powder mixture is granulated by a suitable solvent, the granules are dried, sieved, admixed with a lubricant and compressed to tablets. In the case of direct compression technology generally microcrystalline cellulose having an average particle size of at least 90 µm are used. In the wet granulation process generally microcrystalline cellulose having an average particle size of 50 µm is applied. Recently microcrystalline cellulose containing about 2 % by weight of colloidal silicium dioxide has been used; this microcrystalline cellulose is called as silicified microcrystalline cellulose, and marketed under the name Prosolv.

It has not been described in prior art and it is new that microcrystalline cellulose having an average particle size below 30 µm suspended in a granulating liquid and used for granulation is suitable for the improvement of the tabletting properties of deramciclane-fumarate active ingredient which can generally be compressed to tablets only with high difficulties.

The granules comprising deramciclane-fumarate prepared as described above are thereafter admixed with disintegrating agent(s), lubricant(s), anti-adhesive auxiliary agent(s) and/or filler(s) and the homogenized mixture thus obtained is compressed into tablets.

As disintegrating agent excipients conventionally used in pharmaceutical compositions for such purposes can be used, e.g. various types of starch, preferably corn starch, potato or wheat starch, sodium carboxymethyl starch, sodium carboxymethyl cellulose, lower substituted hydroxypropyl cellulose or crosslinked polyvinylpyrrolidone or a mixture thereof, particularly sodium carboxymethyl cellulose.

The disintegrating agent content of the tablets is 1-10 % by weight, preferably 2-8 % by weight, particularly 3-5 % by weight, related to the total weight of the composition.

As lubricant excipients generally used in pharmaceutical compositions for this purpose can be used, preferably magnesium stearate, calcium stearate, stearic acid, sodium steraril fumarate, hydrogenated vegetable oils or sugar esters, particularly magnesium stearate.

The lubricant content is 0.5-4 % by weight, preferably 0.5-2 % by weight, particularly preferably 1.0-1.5 % by weight, related to the total weight of the tablets.

As anti-adhesive agent excipients generally used in pharmaceutical compositions for this purpose can be used, preferably talc, colloidal and/or micronized silicium dioxide particularly colloidal silicium dioxide.

The amount of the anti-adhesive agent is 0.5-4 % by weight, preferably 0.5-2 % by weight, particularly 1.1-1.5 % by weight, related to the total weight of the tablet.

The pharmaceutical compositions of the present invention contain as filler preferably microcrystalline cellulose or having a particle size of at least 50 µm or silicified microcrystalline cellulose.

The amount of the filler is 0-30 % by weight, preferably 10-30 % by weight, particularly 20-30 % by weight, related to the total amount of the tablets.

According to a further aspect of the present invention there is provided a process for the preparation of deramciclane-fumarate containing tablets which comprises granulating more than 50 % by weight of deramciclane-fumarate (related to the total weight of the tablet) with 5-20 % by weight of microcrystalline cellulose having an average particle size below 30 µm, suspended in a solution of 5-20 % by weight of a binder; homogenizing the granules obtained with 1-15 % by weight of a disintegrant, 0.5-4 % by weight of a lubricant, 0.5-4 % by weight of an anti-adhesive agent and 0-30 % by weight of a binder; and thereafter compressing the mixture to tablets.

According to a preferred form of realization of the process of the present invention the granulation of more than 50 % by weight of deramciclane-fumarate (related to the total weight of the tablet) is carried out with 5-10 % by weight of microcrystalline cellulose having an average particle size below 30 µm, suspended in an aqueous solution of 5-10 % by weight of a binder. The composition of the granulating suspension is determined - similarly to conventionally used granulating liquids - so that in case of high stear granulation the suspensions should still be easily handable, while in case of fluidization spraying granulation it should be suitably sprayable. The determination of the concentration of the binder belongs to the knowledge of the skilled art worker. The amount of microcrystalline cellulose is 5-20 %, related to the total weight of the granulating liquid.

According to a preferred form of realization of the process of the present invention a solution of the binder formed with water, ethanol or isopropanol can be used.

The granulating and tabletting step can be carried out by conventional methods of pharmaceutical industry by using the granulating suspension prepared above.

According to a particularly preferable form of realization of the process of the present invention one can accomplish granulation more than 50 % by weight of deramciclane-fumarate (related to the total weight of the tablet) with 6-9 % by weight of microcrystalline cellulose having an average particle size below 30 µm, suspended in an aqueous solution of 6-9 % by weight of povidone; homogenizing the granules thus obtained with 3-5 % by weight of sodium carboxymethyl cellulose, 1-1.5 % by weight of magnesium stearate, 1-1.5 % by weight of colloidal silicium dioxide and 20-30 % by weight of microcrystalline cellulose having an average particle size above than 50 µm; and compressing the mixture to tablets.

The tablets of the present invention have suitable crushing strength and can be particularly preferably used in film coating process where the mechanical stress is very high. Film coating of the tablets can be carried out by known methods of pharmaceutical industry. Thus for this purpose e.g. film coating compositions marketed under the name Opadry can be used, whereby hydroxypropyl methyl cellulose (Opadry I and Opadry II) or polyvinyl alcohol (Opadry II HP) are applied as film forming polymer.

Further details of the present invention are to be found in the following Examples without limiting the scope of protection to said Examples.

### Example 1

The granulating solution is prepared by dissolving 245 g of co-povidone in 1500 ml of water and thereafter dispersing in the solution 245 g of microcrystalline cellulose type No. 105 having an average particle size below 30 µm.

The granules are prepared by introducing 1470 g of deramciclane-fumarate into the container of a Glatt WSG 1 type fluidizing granulating apparatus, maintaining the active ingredient in fluidized state by air stream having a temperature of 40°C and thereafter spraying the granulating liquid thus obtained onto the powder within about 45 minutes. The granules formed are dried and sieved on a sieve having a hole-size of 1 mm.

To 224 g of the granules thus obtained 24 g of sodium carboxymethyl cellulose disintegrant, 4.0 g of magnesium stearate lubricant and as filler 35 g of Prosolv SMCC 90 (microcrystalline cellulose having an average particle size of 100 µm and containing 2 % by weight of colloidal silicium dioxide) are added. The homogenized mixture thus obtained is compressed on a Fette E XI type press machine into biconvex form tablets weighing 160 mg and having a diameter of 8 mm. The deramciclane base content of the tablets is 60.7 mg; the deramciclane-fumarate content of the tablets amounts to 52.5 % by weight.

During compression no sticking was observed on the surface of punches or the tablets. The internal surface of the broken (crushed) tablets was homogenous and free of lamination.

On measuring the critical parameters of the tablets according to the methods of the European Pharmacopoeia the following results are obtained:

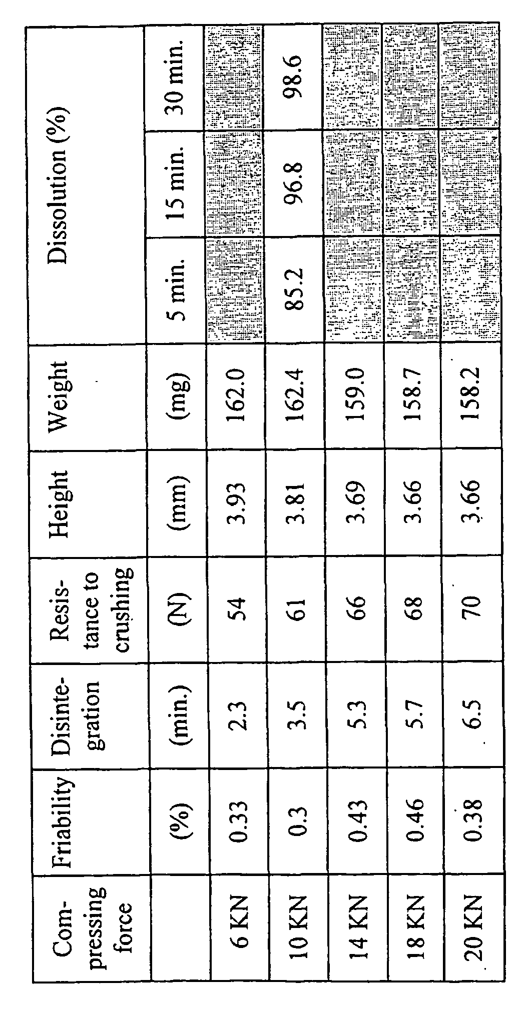

Taking into consideration the requirements of the European Pharmacopoeia the quality of deramciclane-fumarate tablets prepared according to Example 1 is highly favourable.

The requirements of European Pharmacopoeia (Ph. Eur.) are as follows:

| Tablet parameter | | Specification of Ph. Eur. |
|---|---|---|
| Deviation of the weight of the tablet: | | |
| | below 80 mg | ± 10 % |
| | between 80-250 mg | ± 7.5 % |
| | above 250 mg | ± 5 % |
| Active ingredient of the tablet: | | |
| | nominal value. | ± 5 % |
| Deviation of active ingredient content: | | |
| | average value | ± 15% |
| Disintegration time | | max. 15 min. |
| Friability | | max. 1 % |
| Strength of the tablet* | | no specification |
| Dissolution of the active ingredient | | no general |
| | | specification* |

| | | |
|---|---|---|
| *most severe requirement: within 15 minutes more than 85 % of the active ingredient should be dissolved. [Note for guigance on the investigation of bioavailability and bioequivalence, EMEA (European Agency for the Evaluation of Medical Products), 1999]. | | |

### Test methods:

4^{th} European Pharmacopoeia
2.9.1 Disintegration of tablets and capsules
2.9.3 Dissolution test for solid dosage forms
2.9.7 Friability of uncoated tablets
2.9.8 Resistance to crushing of tablets

### Example 2

Granules having the composition shown in the following table are prepared from the granule according to Example 1, whereupon the homogenized mixture is compressed into tablets on a Fette E XI press machine into biconvex form tablets weighing 160 mg and having a diameter of 8 mm. The deramciclane base content of the tablets is 60.7 mg, the deramciclane-fumarate content of the tablets amounts to 52.5 mg.

| No. of experiment | 21. | 22. | 23. | 24. | 25. |
|---|---|---|---|---|---|
| Granules | 224.0 g | 224.0 g | 224.0 g | 224.0 g | 224.0 g |
| Sodium-carboxymethylcellulose | 18.0g | 12.0 g | 12.0 g | 12.0 g | 12.0 g |
| Microcrystalline cellulose (102) | 70.0 g | 66.0 g | 74.0 g | 72.0 g | 70.0 g |
| Magnesium stearate | 5.4 g | 6.0 g | 6.0 g | 6.0 g | 6.0 g |
| Colloidal silicium-dioxide | 2.6 g | 12.0 g | 4.0 g | 6.0 g | 8.0 g |

During compression no sticking was observed on the surface of punches or the tablets. The internal surface of the broken (crushed) tablets was homogenous and free of lamination.

On measuring the critical parameters of the tablets according to the methods of the European Pharmacopoeia the following results are obtained:

| No. of experiment | 21. | | 22. | | 23. | | 24. | | 25. | |
|---|---|---|---|---|---|---|---|---|---|---|
| Compressing force | Resistance to crushing | Disintegration | Resistance to crushing | Disintegration | Resistance to crushing | Disintegration | Resistance to crushing | Disintegration | Resistance to crushing | Disintegration |
| | (N) | (min.) | (N) | (min.) | (N) | (min.) | (N) | (min.) | (N) | (min.) |
| 6 KN | 76.3 | 3.0 | 63.6 | 1.8 | 70.2 | 2.9 | 80.8 | 2.2 | 72.3 | 2.4 |
| 10 KN | 100.2 | 5.0 | 88.4 | 4.2 | 98.4 | 5.8 | 104.2 | 3.6 | 103.7 | 4.2 |
| 14 KN | 107.6 | 5,5 | 106.1 | 4.6 | 121.9 | 6.2 | 121.3 | 5.2 | 119.4 | 6.0 |
| 18 KN | 94.1 | 6.2 | 112.2 | 5.1 | 138.6 | 7.7 | 142.2 | 6.5 | 136.8 | 7.3 |

Taking into the consideration the relevant specifications of the European Pharmacopoeia the quality of the deramciclane-fumarate tablets prepared according to Example 2 is highly favourable.

### Example 3

The granules are prepared by introducing 1470 g of deramciclane-fumarate into the container of a fluidization granulating apparatus type Glatt WSG 1 and keeping the active ingredient in fluidized condition by air stream having a temperature of 40°C. A granulating liquid having a composition shown in the following table is sprayed on the powder. The granules thus obtained are dried and sieved on a 1 mm hole-size sieve.

| No. of experiment | 31. | 32. | 33. | 34. | 35. |
|---|---|---|---|---|---|
| Copovidone | 140.0 g | 192.5 g | 245.0 g | -- | -- |
| Microcrystalline celulose (105) | 140.0 g | 192.5 g | 245.0 g | 200.0 g | 185.0 g |
| Povidone K 30 | -- | -- | -- | 100.0 g | 135.0 g |
| Purified water | 1500 g | 1500 g | 1500 g | 1200 .g | 1000 g |

A homogenized product having the composition disclosed in the following Table is prepared from the above granules. The homogenized mixture is compressed into tablets on a Fette E XI press machine into biconvex form tablets weighing 160 mg and having a diameter of 8 mm. The deramciclane base content of the tablets is 60.7 mg, the deramciclane-fumarate content of the tablets amounts to 52.5 % by weight.

| No. of experiment | 31. | 32. | 33. | 34. | 35. |
|---|---|---|---|---|---|
| Granules | 200.0 g | 212.0 g | 224.0 g | 228.0 g | 238.0 g |
| Sodium-carboxymethyl -cellulose | 12.0 g | 12.0 g | 12.0 g | 14.0 g | 14.0 g |
| Microcrystalline cellulose (102) | 96.0 mg | 84.0 mg | 72.0 mg | 60.0 g | 50.0 g |
| Magnesium stearate | 6.0 g | 6.0 g | 6.0 g | 12.0 g | 12.0 g |
| Colloidal silicium-dioxide | 6.0 g | 6.0 g | 6.0 g | 6.0 g | 6.0 g |

During compression no sticking was observed on the surface of punches or the tablets. The internal surface of the broken (crushed) tablets was homogenous and free of lamination.

On measuring the critical parameters of the tablets according to the methods of the European Pharmacopoeia the following results are obtained:

| No. of experment | 31. | | 32. | | 33. | | 34. | | 35. | |
|---|---|---|---|---|---|---|---|---|---|---|
| Compressing force | Resistance to crushing | Disintegration | Resistance to crushing | Disintegration | Resistance to crushing | Disintegration | Resistance to crushing | Disintegration | Resistance to crushing | Disintegration |
| | (N) | (min.) | (N) | (min.) | (N) | (min.) | (N) | (min.) | (N) | (min.) |
| 6 KN | 34.9 | 0.3 | 43.0 | 1.1 | 48.5 | 1.6 | 42.8 | 1.3 | 47.3 | 1.9 |
| 10 KN | 50.0 | 0.8 | 74.7 | 2.1 | 79.3 | 3.7 | 60.4 | 2.6 | 69.3 | 2.6 |
| 14 KN | 66.5 | 0.9 | 102.. | 4.9 | 112.4 | 5.3 | 63.9 | 3.2 | 64.4 | 3.0 |
| 18 KN | 73.2 | 0.9 | 112.3 | 5.9 | 121.4 | 6.5 | 56.8 | 4.0 | 68.4 | 3.6 |

Taking into the consideration the relevant specifications of the European Pharmacopoeia the quality of the deramciclane-fumarate tablets prepared according to Example 3 is highly favourable.

### Example 4

The granulating solution is prepared by dissolving 175 g of povidone K30 type polyvinyl pyrrolidone in 1000 ml of water and dispersing in the solution 175 g of No. 105 type microcrystalline cellulose having an average particle size below 30 µm.

The granules are prepared by introducing 1453 g of deramciclane-fumarate into the container of a Glatt WSG 1 type fluidizing granulating apparatus and keeping the active ingredient in fluidized condition by air stream having a temperature of 40°C. Thereafter the above granulating liquid is sprayed onto the powder within about 30 minutes. The granules thus obtained are dried and sieved on a 1 mm hole-size sieve.

The granules thus obtained are homogenized into a blend having the composition disclosed in the above table. The blends are compressed on a Fette E XI press machine into biconvex form tablets having a diameter of 8 mm; the active ingredient content of the tablets is always 60 mg deramciclane base. Depending on the composition of the granulating liquid the deramciclane-fumarate content of the tablets varies between 51.8 and 60.3 % by weight.

| No. of experiment | 41. | 42. | 43.* | 44. | 45.* |
|---|---|---|---|---|---|
| Granules | 206.0 g | 206.0 g | 206.0 g | 206.0 g | 206.0 g |
| Sodium-carboxymethyl -cellulose | 12.0 g | 6.0 g | -- | 12.0 g | 12.0 g |
| Microcrystalline cellulose (102) | 90.0 mg | 90.0 mg | 90.0 mg | 45.0 g | -- |
| Magnesium stearate | 6.0 g | 6.0 g | 6.0 g | 6.0 g | 6.0 g |
| Colloidal silicium dioxide | 6.0 g | 6.0 g | 6.0 g | 6.0 g | 6.0 g |

During compression no sticking was observed on the surface of punches or the tablets. The internal surface of the broken (crushed) tablets was homogenous and free of lamination.

The critical parameters of the tablets were determined in accordance with the corresponding specification of the European Pharmacopoeia. The results obtained are summarized in the following Table:

| No. of experiment | 41. | | 42. | | 43. | | 44. | | 45. | |
|---|---|---|---|---|---|---|---|---|---|---|
| Compressing force | Resistance to crushing | Disintegration | Resistance to crushing | Disintegration | Resistance to crushing | Disintegration | Resistance to crushing | Disintegration | Resistance to crushing | Disintegration |
| | (N) | (min.) | (N) | (min.) | (N) | (min.) | (N) | (min.) | (N) | (min.) |
| 6 KN | 51.8 | 1.5 | 48.4 | 1.3 | 53.7 | 4.3 | 26.7 | 1.6 | 23.8 | 2.1 1 |
| 10 KN | 703 | 2.3 | 64.8 | 2.5 | 55.9 | 7.3 | 46.9 | 2.4 | 24.1 | 2.4 |
| 14 KN | 75.3 | 2.5 | 69.3. | 3.3 | 58.1 | 8.1 | 49.3 | 2.8 | 29.0 | 2.6 |
| 18 KN | 78.2 | 3.0 | 72.4 | 4.9 | 60.2 | 9.7 | 48.5 | 3.1 | 23.1 1 | 2.6 |

| | | | | | | | | | | |
|---|---|---|---|---|---|---|---|---|---|---|
| *Experiments 43 and 45 are of comparative character. The composition No. 43 contains no disintegrating agent and therefore the disintegration time of the tablets is significantly longer. Contrary to the present invention composition No. 45 contains no microcrystalline cellulose having an average particle size of at least 50 µm and the strength of the tablets does not reach the required crushing strength of at least 40 N. | | | | | | | | | | |

### Example 5

The granulating solution is prepared by dissolving 245 g of povidone K30 type polyvinyl pyrrolidone in 1500 ml of water and dispersing in the solution 245 g of type No. 105 microcrystalline cellulose having an average particle size below 30 µm.

The granules are prepared by introducing 1470 g of deramciclane-fumarate into the container of a Glatt WSG 1 type fluidizing granulating apparatus and keeping the active ingredient in fluidized state by air stream having a temperature of 40°C. The granulating liquid thus obtained is sprayed onto the powder within about 30 minutes. The granules thus formed are dried and sieved on a 1 mm hole-size sieve.

The granules thus obtained are admixed with 105 g of sodium carboxymethyl cellulose, 52.5 g of magnesium stearate, 52.5 g of colloidal silicium dioxide and 630 g of microcrystalline cellulose (No. 102, average particle size 90 µm). The blend is compressed into biconvex form tablets weighing 160 mg and having a diameter of 8 mm on a Fette E XI eccentric press machine or a Manesty Betapress rotating press machine under 55 r.p.m. The deramciclane base content of the tablets is 60.7 mg, the deramciclane-fumarate content of the tablets is 52.5 % by weight.

During compression no sticking was observed on the surface of punches or the tablets. The internal surface of the broken (crushed) tablets was homogenous and free of lamination.

On measuring the critical parameters of the tablets according to the methods of the European Pharmacopoeia the following results are obtained:

| No. of experiment | 51. | | 52. | |
|---|---|---|---|---|
| | Fette E XI eccentric press machine | | Betapress rotating press machine | |
| Compressing force | Resistance to crushing | Disintegration time | Resistance to crushing | Disintegration time |
| | (N) | (min.) | (N) | (min.) |
| 6 KN | 50.3 | 2.0 | -- | -- |
| 10 KN | 81.6 | 4.1 | 84.5 | 4.6 |
| 14 KN | 99.4 | 5.4 | 104.5 | 5.5 |
| 18 KN | 112.2 | 6.5 | -- | -- |

The compressability of the granules is suitable and not dependent on the type of the press machine used.

### Example 6

The granulating solution is prepared by dissolving 600 g of povidone K30 type polyvinyl pyrrolidone in 3.6 kg of water and dispersing in the solution 600 g of microcrystalline cellulose type No. 105 having an average particle size below 30 µm.

The granules are prepared by introducing 4.98 g of deramciclane-fumarate into the container of a Glatt WSG 5 type fluidizing granulating apparatus and keeping the active ingredient in fluidized state by air stream having a temperature of 60°C. The granulating solution is sprayed on the powder within about 60 minutes. The granules formed are dried and sieved on a 1 mm hole-size sieve.

The granules thus obtained are admixed with 360 g of sodium carboxymethyl cellulose, 180 g of magnesium stearate, 180 g of colloidal silicium dioxide and 2.70 kg of microcrystalline cellulose (No. 102 having an average particle size of 90 µm). The blend is compressed on a Manesty Betapress rotating press machine into biconvex formed tablets weighing 80 mg and having a diameter of 6 mm, or weighing 160 mg and having a diameter of 8 mm respectively. The deramciclane base content of the tablets is 30 mg/tablet and 60 mg/tablet respectively, the deramciclane-fumarate content of the tablets amounts to 51.8 % by weight.

During compression no sticking was observed on the surface of punches or the tablets. The internal surface of the broken (crushed) tablets was homogenous and free of lamination.

On measuring the critical parameters of the tablets according to the methods of the European Pharmacopoeia the following results are obtained:

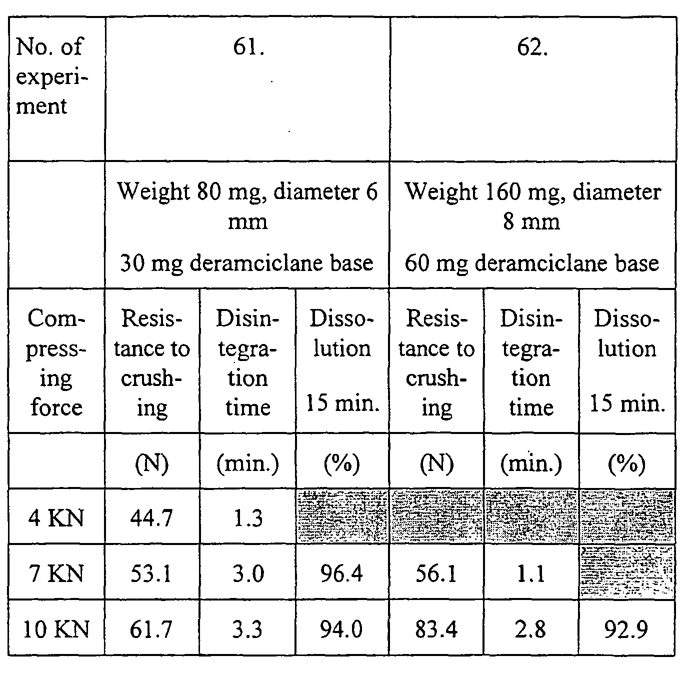

In view of the relevant specifications of the European Pharmacopoeia the quality of the deramciclane-fumarate tablets thus obtained is highly favourable. On the basis of the mechanical characteristics the tablets are suitable for filmcoating.

### Example 7

The granulating solution is prepared by dissolving 2.0 kg of povidone K30 type polyvinyl pyrrolidone in 12 kg of water and dispersing in the solution 2.0 kg of No. 105 microcrystalline cellulose having an average particle size below 30 µm.

The granules are prepared by introducing 16.61 kg of deramciclane-fumarate into the container of a Glatt GPCG 15 type fluidizing granulating apparatus and keeping the active ingredient in fluidized state by air stream having a temperature of 60°C. The granulating solution thus obtained is sprayed on the powder within about 80 minutes. The granules thus formed are dried and sieved on a 1 mm hole-size sieve.

The granules thus obtained are admixed with 1.2 kg of sodium carboxymethyl cellulose, 0.40 kg of magnesium stearate, 0.40 kg of colloidal silicium dioxide and 8.99 kg of microcrystalline cellulose (No. 102, an average particle size of 90 µm). The blend is compressed on a Manesty Betapress rotating press machine under 60 r.p.m. into biconvex formed tablets weighing 80 mg, having a diameter of 6 mm and weighing 160 mg, having a diameter of 8 mm, respectively. The deramciclane base content of the tablets amount to 30 mg/tablet and 60 mg/tablet, respectively. The deramciclane-fumarate content of the tablets is 51.8 % by weight.

During compression no sticking was observed on the surface of punches or the tablets. The internal surface of the broken (crushed) tablets was homogenous and free of lamination.

On measuring the critical parameters of the tablets according to the methods of the European Pharmacopoeia the following results are obtained:

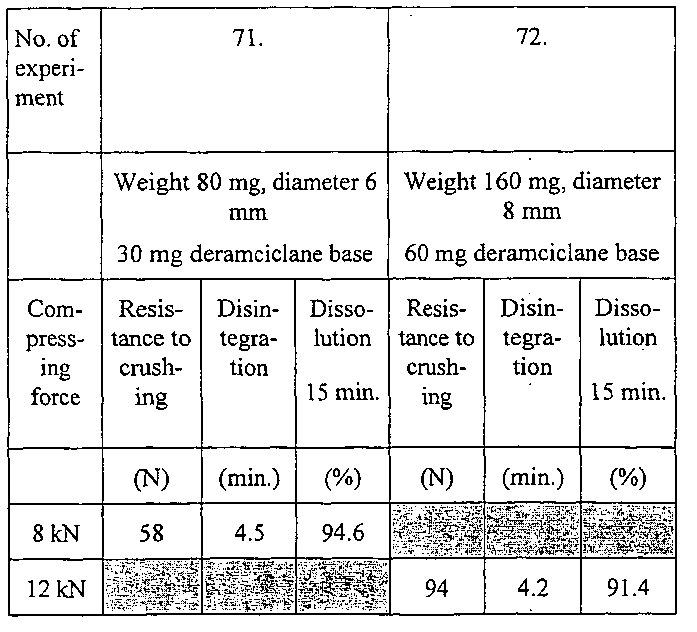

In view of the relevant specifications of the European Pharmacopoeia the quality of the deramciclane-fumarate tablets thus prepared is highly favourable. On the basis of the mechanical characteristics the tablets are suitable for filmcoating.

### Example 8 (comparative example)

As a comparison deramciclane tablets are prepared by omitting in the granulating liquid according to Example 5 the microcrystalline cellulose type No. 105 (particle size below 30 µm) and replacing it by increasing the amount of microcrystalline cellulose by adding No. 102 type microcrystalline cellulose (particle size 90 µm) to the granules.

The granules are prepared by introducing 1470 g of deramciclane-fumarate into the container of a Glatt WSG 1 type fluidizing granulating apparatus and keeping the active ingredient in fluidized state by air stream having a temperature of 40°C. A solution of 245 g povidone K30 type polyvinyl pyrrolidone formed with 1500 ml of water is sprayed onto the powder within about 30 minutes. The granules thus formed are dried and sieved on a 1 mm hole-size sieve.

The granules thus obtained are admixed with 105 g of sodium carboxymethyl cellulose, 52.5 g of magnesium stearate, 52.5 g of colloidal silicium dioxide and 875 g of microcrystalline cellulose (No. 102, an average particle size 90 µm). The blend is compressed on a Fette E XI eccentric press machine or a Manesty Betapress rotating press machine under 55 r.pm. into biconvex formed tablets weighing 160 mg and having a diameter of 8 mm. The deramciclane base content of the tablets is 60.7 mg, the deramciclane-fumarate content of the tablets amounts to 52.5 % by weight.

During the compression of tablets a layer is formed on the pressing tool, the surface of the tablets became cloudy; the quality of the tablets is unsatisfactory and the tablets are unsuitable for industrial scale manufacture.

## Claims

1. Tablets comprising deramciclane-fumarate of the Formula 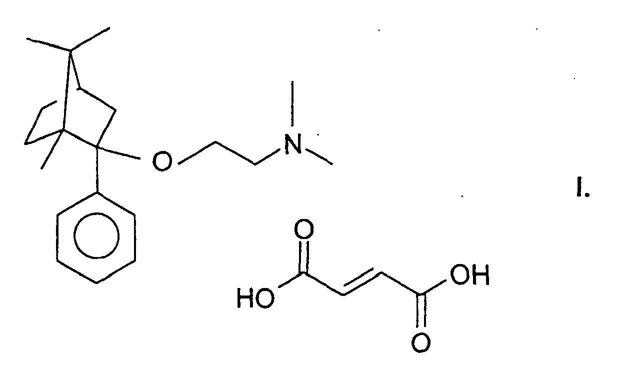 whereby said tablets contain (related to the total weight) more than 50 % by weight of deramciclane-fumarate, 5-20 % by weight of a binder, 5-20 % by weight of microcrystalline cellulose having an average particle size below 30 µm, 1-10 % by weight of a disintegrant, 0.5-4 % by weight of a lubricant, 0.5-4 % by weight of an anti-adhesive agent and 0-30 % by weight of a filler.

2. Tablets according to Claim 1 comprising 50-88 % by weight, preferably 50-78 % by weight of deramciclane-fumarate.

3. Tablets according to Claim 1 comprising as binder polyvinyl pyrrolidone, gum-arabic, alginic acid, sodium carboxymethyl cellulose, dextrine, ethyl cellulose; gelatine, liquid sugar, guar gum, hydroxypropyl methyl cellulose, methyl cellulose, polyethylene oxide, pre-gelatinised starch or sugar syrup.

4. Tablets according to Claim 3 comprising as binder polyvinyl pyrrolidone.

5. Tablets according to Claim 3 or 4 comprising 5-12 % by weight of a binder.

6. Tablets according to Claim 5 comprising 6-9 % by weight of a binder.

7. Tablets according to Claim 1 comprising 5-15 % by weight of microcrystalline cellulose having an average particle size below 30 µm.

8. Tablets according to Claim 7 comprising 6-9 % by weight of microcrystalline cellulose having an average particle size below 30 µm.

9. Tablets according to Claim 1 comprising as disintegrant starch, preferably maize, potato or wheat starch, sodium carboxymethyl starch, sodium carboxymethyl cellulose, lower substituted hydroxypropyl cellulose or crosslinked polyvinyl pyrrolidone or a mixture thereof.

10. Tablets according to Claim 9 comprising as disintegrant sodium carboxymethyl cellulose.

11. Tablets according to Claim 9 or 10 comprising 2-8 % by weight of a disintegrant.

12. Tablets according to Claim 11 comprising 3-5 % by weight of said disintegrant.

13. Tablets according to Claim 1 comprising as lubricant magnesium stearate, calcium stearate, stearic acid, sodium stearil fumarate, hydrogenated vegetable oils or sugar esters.

14. Tablets according to Claim 13 comprising as lubricant magnesium stearate.

15. Tablets according to Claim 13 or 14 comprising said lubricant in an amount of 0.5-2 % by weight.

16. Tablets according to Claim15 comprising said lubricant in an amount of 1.0-1.5 % by weight.

17. Tablets according to Claim 1 comprising as anti-adhesive agent colloidal silicium dioxide.

18. Tablets according to Claim 17 comprising colloidal silicium dioxide in an amount of 0.5-2 % by weight.

19. Tablets according to Claim 18 comprising colloidal silicium dioxide in an amount of 1.0-1.5 % by weight.

20. Tablets according to Claim 1 comprising as filler microcrystalline cellulose having a particle size of at least 50 µm or microcrystalline cellulose containing colloidal silicium dioxide.

21. Tablets according to Claim 20 comprising said filler in an amount of 10-30 % by weight.

22. Tablets according to Claim 21 comprising said filler in an amount of 20-30 % by weight.

23. Tablets according to Claim 1 comprising as active ingredient (1R,2S,4R)-(-)-2-dimethylaminoethoxy-2=phenyl-1,7,7-trimethyl-bicyclo[2.2.1]heptane-2-(E)-butenedioate (1:1) of the Formula I which contains not more than 0.2 % by weight of (1R,3 S,4R)-(-)-3-(2-N,N-dimethylaminoethyl)-1,7,7-trimethylbicyclo[2.2.1]heptane-2-one-(E)-butenedioate (1:1) of the Formula

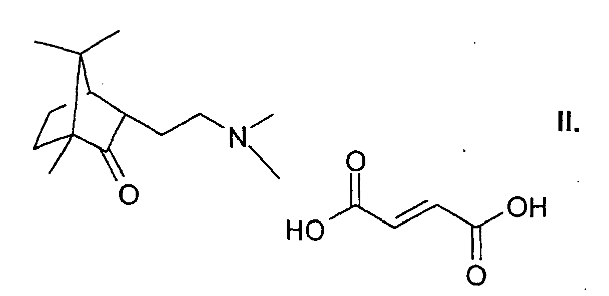

24. Process for the preparation of deramciclane-fumarate containing tablets according to Claim 1 which comprises granulating more than 50 % by weight of the deramciclane-fumarate (related to the total weight of the tablet) with 5-20 % by weight of microcrystalline cellulose having an average particle size below 30 µm, suspended in a solution of 5-20 % by weight of a binder; homogenizing the granules obtained with 1-15 % by weight of a disintegrant, 0.5-4 % by weight of a lubricant, 0.5-4 % by weight of an anti-adhesive agent and 0.30 % by weight of a binder; and thereafter compressing the mixture to tablets.

25. Process according to Claim 24 which comprises using a solution of a binder formed with water, ethanol or isopropanol.

26. Process according to Claim 23 or 24 which comprises granulating more than 50 % by weight of deramciclane-fumarate (related to the total weight of the tablet) with 6-9 % by weight of microcrystalline cellulose having an average particle size below 30 µm, suspended in an aqueous solution of 6-9 % by weight of povidone; homogenizing the granules thus obtained with 3-5 % by weight of sodium carboxymethyl cellulose, 1-1.5 % by weight of magnesium stearate, 1-1.5 % by weight of colloidal silicium dioxide and 20-30 % by weight of microcrystalline cellulose having an average particle size above than 50 µm; and compressing the mixture to tablets.

## Patentansprüche

1. Tabletten, umfassend Deramciclan-fumarat der Formel 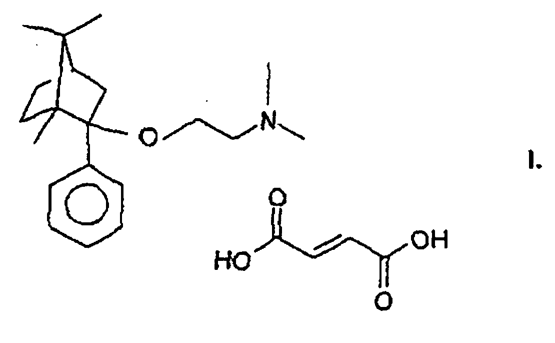 wobei die Tabletten mehr als 50 Gew.-% an Deramciclan-fumarat, 5-20 Gew.-% eines Bindemittels, 5-20 Gew.-% mikrokristalline Cellulose mit einer durchschnittlichen Teilchengröße unter 30 µm, 1-10 Gew.-% eines Zerfallsmittels, 0,5-4 Gew.-% eines Gleitmittels, 0,5-4 Gew.-% eines Antihaftmittels und 0-30 Gew.-% eines Füllstoffes enthalten (bezogen auf das Gesamtgewicht).

2. Tabletten gemäß Anspruch 1, umfassend 50-88 Gew.-%, vorzugsweise 50-78 Gew.-%, an Deramciclan-fumarat.

3. Tabletten gemäß Anspruch 1, umfassend als Bindemittel Polyvinylpyrrolidon, Gummiarabikum, Alginsäure, Natriumcarboxymethylcellulose, Dextrin, Ethylcellulose, Gelantine, flüssigen Zucker, Guar Gummi, Hydroxypropylmethylcellulose, Methylcellulose, Polyethylenoxid, vor-gelantinierte Stärke oder Zuckersirup.

4. Tabletten gemäß Anspruch 3, umfassend als Bindemittel Polyvinylpyrrolidon.

5. Tabletten gemäß Anspruch 3 oder 4, umfassend 5-12 Gew.-% eines Bindemittels.

6. Tabletten gemäß Anspruch 5, umfassend 6-9 Gew.-% eines Bindemittels.

7. Tabletten gemäß Anspruch 1, umfassend 5-15 Gew.-% an mikrokristalliner Cellulose mit einer durchschnittlichen Teilchengröße unter 30 µm.

8. Tabletten gemäß Anspruch 7, umfassend 6-9 Gew.-% an mikrokristalliner Cellulose mit einer durchschnittlichen Teilchengröße unter 30 µm.

9. Tabletten gemäß Anspruch 1, umfassend als Zerfallsmittel Stärke, vorzugsweise Mais-, Kartoffel- oder Weizenstärke, Natriumcarboxymethylstärke, Natriumcarboxymethylcellulose, niedriger substituierte Hydroxypropylcellulose oder vernetztes Polyvinylpyrrolidon oder eine Mischung davon.

10. Tabletten gemäß Anspruch 9, umfassend als Zerfallsmittel Natriumcarboxymethylcellulose.

11. Tabletten gemäß Anspruch 9 oder 10, umfassend 2-8 Gew.-% eines Zerfallsmittels.

12. Tabletten gemäß Anspruch 11, umfassend 3-5 Gew.-% des Zerfallsmittels.

13. Tabletten gemäß Anspruch 1, umfassend als Gleitmittel Magnesiumstearat, Calciumstearat, Stearinsäure, Natriumstearylfumarat, hydrierte Pflanzenöle oder Zuckerester.

14. Tabletten gemäß Anspruch 13, umfassend als Gleitmittel Magnesiumstearat.

15. Tabletten gemäß Anspruch 13 oder 14, umfassend das Gleitmittel in einer Menge von 0,5-2 Gew.-%.

16. Tabletten gemäß Anspruch 15, umfassend umfassend das Gleitmittel in einer Menge von 1,0-1,5 Gew.-%.

17. Tabletten gemäß Anspruch 1, umfassend als Antihaftmittel kolloidales Silciumdioxid.

18. Tabletten gemäß Anspruch 17, umfassend kolloidales Silciumdioxid in einer Menge von 0,5-2 Gew.-%.

19. Tabletten gemäß Anspruch 18, umfassend kolloidales Silciumdioxid in einer Menge von 1,0-1,5 Gew.-%.

20. Tabletten gemäß Anspruch 1, umfassend als Füllstoff mikrokristalline Cellulose mit einer Teilchengröße von mindestens 50 µm oder mikrokristalline Cellulose, die kolloidales Silciumdioxid enthält.

21. Tabletten gemäß Anspruch 20, umfassend den Füllstoff in einer Menge von 10-30 Gew.-%.

22. Tabletten gemäß Anspruch 21, umfassend den Füllstoff in einer Menge von 20-30 Gew.-%.

23. Tabletten gemäß Anspruch 1, umfassend als aktiven Bestandteilen (1R, 2S, 4R)-(-)-2-Dimethylaminoethoxy-2-phenyl-1,7,7-trimethyl-bicyclo[2.2.1]hepta-2-(E)-butendioat (1:1) der Formel I, welche nicht mehr als 0,2 Gew.-% an (1R,3S,4R)-(-)-3-(2-N,N-Dimethylaminoethyl)-1,7,7-trimethyl-bicyclo[2.2.1]hepta-2-on-(E)-butendioat (1:1) der Formel 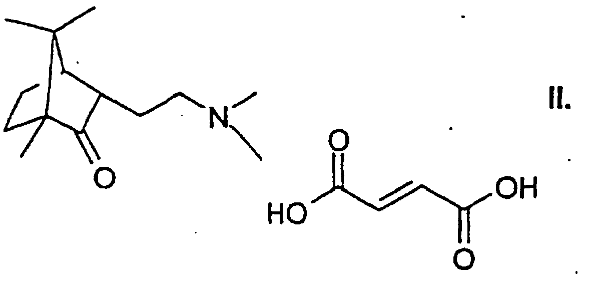 enthalten.

24. Verfahren zur Herstellung von Deramciclan-fumarat enthaltenden Tabletten gemäß Anspruch 1, welches das Granulieren von mehr als 50 Gew.-% des Deramciclan-fumarats (in Bezug auf das Gesamtgewicht der Tabletten) mit 5-20 Gew.-% an mikrokristalliner Cellulose mit einer durchschnittlichen Teilchengröße von unter 30 µm, suspendiert in einer Lösung von 5-20 Gew.-% eines Bindemittels; das Homogenisieren der erhaltenen Granulate mit 1-15 Gew.-% eines Zerfallsmittels, 0,5-4 Gew.-% eines Gleitmittels, 0,5-4 Gew.-% eines Antihaftmittels und 0,30 Gew.-% eines Bindemittels; und danach das Pressen der Mischung zu Tabletten umfasst.

25. Verfahren gemäß Anspruch 24, welches die Anwendung einer Lösung eines Bindemittels, gebildet mit Wasser, Ethanol oder Isopropanol, umfasst.

26. Verfahren gemäß Anspruch 23 oder 24 welches das Granulieren von mehr als 50 Gew.-% an Deramciclan-fumarat (in Bezug auf das Gesamtgewicht der Tablette) mit 6-9 Gew.-% an mikrokristalliner Cellulose mit einer durchschnittlichen Teilchengröße unter 30 µm, suspendiert in einer wässrigen Lösung von 6-9 Gew.-% an Povidon; das Homogenisieren der so erhaltenen Granulate mit 3-5 Gew.-% an Natriumcarboxymethylcellulose, 1-1,5, Gew.-% Magnesiumstearat, 1-1,5, Gew.-% an kolloidalem Silciumdioxid und 20-30 Gew.-% an mikrokristalliner Cellulose mit einer durchschnittlichen Teilchengröße von über 50 µm; und das Pressen der Mischung zu Tabletten umfasst.

## Revendications

1. Les comprimés sont constitués de déramciclane-fumarate de Formule 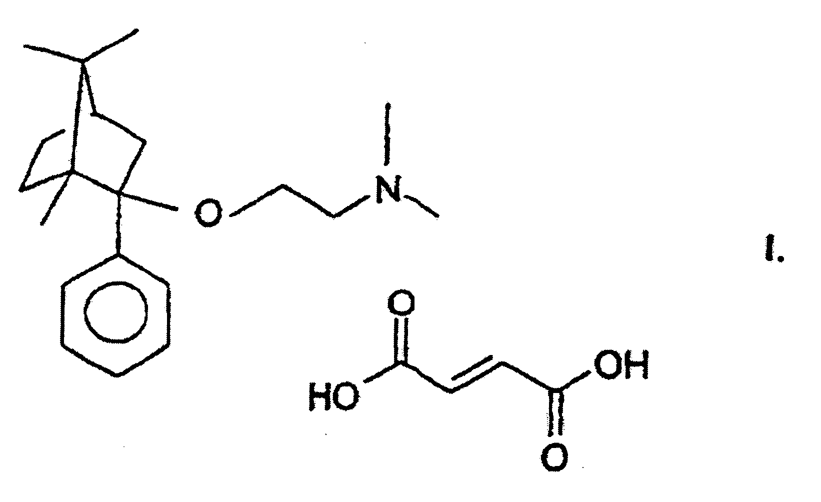 où les dits comprimés contiennent (par rapport au poids total) plus de 50% en poids de déramciclane-fumarate, de 5 à 20% en poids de liant, de 5 à 20% en poids de cellulose microcristalline ayant une taille de particule moyenne se situant en deçà de 30 µm, de 1 à 10% en poids d'agent de désagrégation, de 0.5 à 4% en poids d'agent lubrifiant, de 0.5 à 4% en poids d'agent anti-adhésif et de 0 à 30% en poids de matière charge.

2. Les comprimés selon la Revendication 1 contiennent de 50 à 88% en poids, de préférence 50 à 78% en poids de déramciclane-fumarate.

3. Les comprimés selon la Revendication 1 contiennent un liant comme du polyvinylpyrrolidone, de la gomme arabique, de l'acide alginique, du carboxyméthyl cellulose sodique, de la dextrine, de l'éthyl cellulose, de la gélatine, du sucre liquide, de la gomme de guar, de l'hydroxypropyl méthyl cellulose, de la méthyle cellulose, de l'oxyde polyéthylène, de l'amidon prégélatinisé ou du sirop de sucre.

4. Les comprimés selon la Revendication 3 ont pour liant du polyvinylpyrrolidone.

5. Les comprimés selon la Revendication 3 ou 4 contiennent de 5 à 12% en poids de liant.

6. Les comprimés selon la Revendication 5 contiennent de 6 à 9% en poids de liant.

7. Les comprimés selon la Revendication 1 contiennent de 5 à 15% en poids de cellulose microcristalline ayant une taille de particule moyenne se situant en deçà de 30µm.

8. Les comprimés selon la Revendication 7 contiennent de 6 à 9% en poids de cellulose microcristalline ayant une taille de particule moyenne se situant en deçà de 30µm.

9. Les comprimés selon la Revendication 1 contiennent un agent de désagrégation comme l'amidon, de préférence de l'amidon de maïs, de pomme de terre ou de blé, de sodium carboxyméthylamidon, de carboxyméthyl cellulose sodique, d'hydroxypropyl cellulose ayant une substitution plus faible ou de polyvinylpyrrolidone réticulé ou un mélange de ceux-ci.

10. Les comprimés selon la Revendication 9 contiennent de la carboxyméthyl cellulose sodique comme agent de désagrégation.

11. Les comprimés selon la Revendication 9 ou 10 contiennent de 2 à 8% en poids d'agent de désagrégation.

12. Les comprimés selon la Revendication 11 contiennent de 3 à 5% en poids du dit agent de désagrégation.

13. Les comprimés selon la Revendication 1 contiennent un agent lubrifiant comme le stéarate de magnésium, le stéarate de calcium, l'acide stéarique, le sodium stearyl fumarate, l'huile végétales hydrogénées ou des esters de sucre.

14. Les comprimés selon la Revendication 13 contiennent un agent lubrifiant comme le stéarate de magnésium.

15. Les comprimés selon la Revendication 13 ou 14 contiennent le dit agent lubrifiant dans une quantité de 0.5 à 2% en poids.

16. Les comprimés selon la Revendication 15 contiennent le dit agent lubrifiant dans une quantité de 1.0 à 1.5% en poids.

17. Les comprimés selon la Revendication 1 contiennent du dioxyde de silicium colloïdal comme agent anti-adhésif.

18. Les comprimés selon la Revendication 17 contiennent du dioxyde de silicium colloïdal dans une quantité de 0.5 à 2% en poids.

19. Les comprimés selon la Revendication 18 contiennent du dioxyde de silicium colloïdal dans une quantité de 1.0 à 1.5% en poids.

20. Les comprimés selon la Revendication 1 sont constitués de cellulose microcristalline comme matière charge ayant une taille de particule d'au moins 50 µm ou de cellulose microcristalline contenant du dioxyde de silicium colloïdal.

21. Les comprimés selon la Revendication 20 contiennent la dite matière charge dans une quantité de 10 à 30% en poids.

22. Les comprimés selon la Revendication 21 contiennent la dite matière charge dans une quantité de 20 à 30% en poids.

23. Les comprimés selon la Revendication 1 ont pour ingrédient actif (1R, 2S, 4R)-(-)-2-diméthylamino éthoxy-2-phenyl-1,7,7-triméthyl-bicyclo[2.2.1]heptane-2-(E)-butènedioate(1:1) de Formule I ne contenant pas plus de 0.2% en poids de (1R, 3S, 4R)-(-)-3-(2-N, N-diméthylaminoéthyl)-1,7,7-triméthyl-bicyclo[2.2.1]heptane-2-one-(E)butènedioate(1:1) de Formule

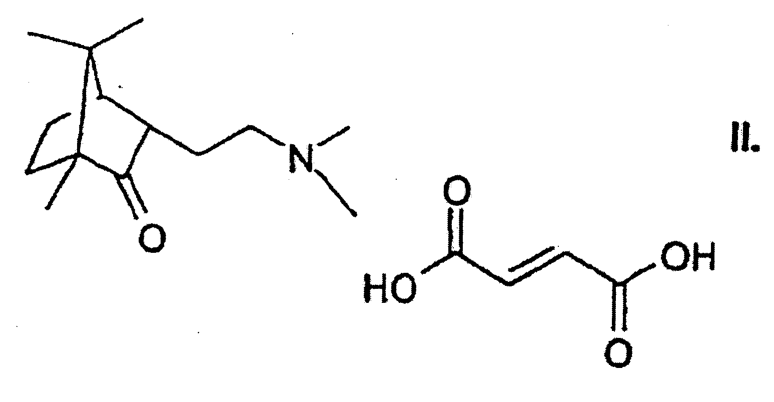

24. Le procédé de préparation des comprimés contenant du déramciclane-fumarate selon la Revendication 1 qui comprend une granulation de plus de 50% en poids de déramciclane-fumarate (par rapport au poids total du comprimé) avec de 5 à 20% en poids de cellulose microcristalline ayant une taille de particule moyenne se situant en deçà de 30µm, en suspension dans une solution de 5 à 20% en poids du liant ; l'homogénéisation des granules obtenues avec de 1 à 15% en poids d'agent de désagrégation, de 0.5 à 4% en poids d'agent lubrifiant, de 0.5 à 4% en poids d'agent anti-adhésif et 0.30% en poids du liant ; et comprimer ensuite ce mélange pour obtenir des comprimés.

25. Le procédé selon la Revendication 24 qui comprend l'utilisation d'une solution d'un liant formée avec de l'eau, de l'éthanol ou de l'isopropanol.

26. Le procédé selon la Revendication 23 ou 24 qui comprend la granulation de plus de 50% en poids de déramciclane-fumarate (par rapport au poids total du comprimé) avec 6 à 9% en poids de cellulose microcristalline ayant une taille de particule moyenne se situant en deçà de 30µm, en suspension dans une solution aqueuse de 6 à 9% en poids de povidone ; l'homogénéisation des granules ainsi obtenues avec 3 à 5% en poids de carboxyméthyl cellulose sodique, de 1 à 1.5% en poids de stéarate de magnésium, de 1 à 1.5% en poids de dioxyde de silicium colloïdal et de 20 à 30% en poids de cellulose microcristalline ayant une taille de particule moyenne se situant au-dessus de 50µm ; et comprimer ensuite ce mélange pour obtenir des comprimés.
